# EUROPÄISCHE PATENTSCHRIFT

(11) **EP 4 384 142 B1**
(45) Veröffentlichungstag und Bekanntmachung des Hinweises auf die Patenterteilung: **07.05.2025**
(21) Anmeldenummer: 22735849.6
(22) Anmeldetag: 21.06.2022
(51) Int. Cl.: A61K 8/9789, A61Q 5/06

(54) **BEIZMITTEL-FREIES HAARFÄRBEMITTEL MIT PFLANZENFARBSTOFFEN**
MORDANT-FREE HAIR DYE CONTAINING PLANT DYES
COLORANT CAPILLAIRE SANS MORDANT CONTENANT DES COLORANTS VÉGÉTAUX

(30) Priorität: 13.08.2021 DE 102021208915
(43) Veröffentlichungstag der Anmeldung: 19.06.2024
(73) Patentinhaber: Henkel AG & Co. KGaA, 40589 Düsseldorf (DE)
(72) Erfinder: SARGSYAN, Lusine, 21629 Neu Wulmstorf (DE); MANNECK, Hartmut, 23858 Barnitz (DE); HIPPE, Thomas, 25482 Appen (DE); VILL, Volkmar, 20146 Hamburg (DE)
(86) Internationale Anmeldenummer: PCT/EP2022/066792
(87) Internationale Veröffentlichungsnummer: WO 2023/016685

(56) Entgegenhaltungen:
- WO-A1-2011/141460
- WO-A1-2019/034770
- WO-A1-2020/127441
- DATABASE GNPD [online] MINTEL; 28 May 2021 (2021-05-28), ANONYMOUS: "Coloring Care", XP055978802, retrieved from https://www.gnpd.com/sinatra/recordpage/8737131/ Database accession no. 8737131

## Beschreibung

Die vorliegende Erfindung betrifft eine kosmetische Zusammensetzung zur Oxidationsmittel-freien und Beizmittel-freien Färbung von keratinischen Fasern, insbesondere menschlichen Haaren, unter Verwendung ausgewählter Naturfarbstoffe in einer wässrigen Zusammensetzung, die auf einen pH-Wert im Bereich von 3 - 5,9 gepuffert ist. Die vorliegende Erfindung betrifft weiterhin ein Verfahren zur Färbung keratinhaltiger Fasern, insbesondere menschlicher Haare, unter Verwendung ausgewählter Naturfarbstoffe in einer wässrigen Zusammensetzung.

### Stand der Technik

Der Wunsch, die eigene Haarfarbe zu verändern, ist ein großes Bedürfnis vieler Verbraucher. Um dieses Bedürfnis zu befriedigen, stellt die kosmetische Industrie eine vielfältige Produktpalette bereit. Haarfärbemittel, die eine besonders langanhaltende Färbung mit hoher Deckkraft erzielen, sind meist Oxidationsfärbemittel, siehe beispielsweise DE102011085416A1. Diese setzen Oxidations- und Alkalisierungsmittel ein, die die Haarstruktur schädigen können. Bestimmte kationische Direktfarbstoffe sind ebenfalls in der Lage, Haarfarbveränderungen mit hoher Farbintensität und Chroma und hervorragenden Echtheitseigenschaften zu ermöglichen, siehe beispielsweise EP850638A1. Die genannten Färbemittel enthalten synthetische Farbstoffe.

Bei einer wachsenden Zahl an Verbrauchern besteht der Wunsch nach Haarfärbemitteln und Haarfärbeverfahren, die auf Naturfarbstoffen beruhen, auch wenn diese Mittel und Verfahren den vorgenannten Mitteln und Verfahren an Echtheitseigenschaften, Deckkraft, Farbintensität und Farbenvielfalt unterlegen sind, siehe beispielsweise WO2010063533A1.

Neben dem Färben mit Henna, das aus der Pflanze *Lawsonia inermis* gewonnen wird, ist auch die Färbung der Haare mit bestimmten Polyphenolen, insbesondere mit Tanninen und Pseudotanninen, bereits seit langem bekannt, siehe beispielsweise EP2438900A1 und WO2019/034770A1. Derartige Polyphenole werden aus bestimmten Pflanzenteilen extrahiert. Es ist aber auch möglich, pulverisierte Pflanzenteile mit hohem Polyphenolgehalt im Färbemittel einzusetzen, siehe zum Beispiel DE19905707A1. Daneben ist es möglich, reine Polyphenole, insbesondere Tanninsäure, erfindungsgemäß zu verwenden. Die Haftung des Polyphenol-Farbstoffs auf dem Haar bzw. auf den Keratinfasern und damit die Echtheit der Färbung sollen durch bestimmte zwei- oder dreiwertige Metallsalze positiv beeinflusst werden.

JP2001270812A offenbart ein zweistufiges Färbeverfahren unter Einsatz einer ersten Zusammensetzung, die einen Tannin-haltigen Pflanzenfarbstoff und Natriumsulfit enthält, und einer zweiten Zusammensetzung, die ein Fe(II)-Salz mit einem anorganischen Gegenion enthält, wobei die Keratinfasern zwischen der Applikation der beiden Zusammensetzungen ausgespült werden. Die Offenlegungsschrift WO2010094207A1 offenbart ein Verfahren zum Färben von Haaren, bei dem das Haar zunächst mit einem Keratinreduktionsmittel behandelt und dadurch die Haarstruktur aufgebrochen und geschwächt wird, anschließend eine Zusammensetzung, die einen Tannin-haltigen Pflanzenfarbstoff, Natriumsulfit, Cysteinhydrochlorid und ein Fe(II)-Salz mit einem anorganischen Gegenion enthält, auf das Haar aufgetragen wird.

Mehrstufige Färbeverfahren sind für den Anwender zeitintensiv und daher gegenüber Einschritt-Verfahren weniger bevorzugt. Auch Vorbehandlungen, die die Haarstruktur beeinflussen, um das Haar dadurch auf die Farbstoffaufnahme vorzubereiten, sind zeitintensiv und daher für den Anwender weniger attraktiv. Außerdem müssen für ein Mehrschritt-Verfahren mehrere Zusammensetzungen bereitgestellt werden, was zu einem höheren Verpackungsaufwand für das gesamte Färbemittel führt. Dies ist aus Gründen des Umweltschutzes weniger bevorzugt. Bei Verwendung von Beizmitteln erzielt man dunkle Färbungen. Helle Farbtöne mit zufriedenstellender Waschechtheit sind in einem Einstufenverfahren bislang nicht erhältlich.

### Aufgabe

Der vorliegenden Erfindung lag die Aufgabe zu Grunde, Färbezusammensetzungen für ein Einschritt-Verfahren zur Färbung keratinhaltiger Fasern, insbesondere menschlicher Haare, bereitzustellen, das helle Farbtöne mit guten Echtheitseigenschaften, insbesondere mit einer hohen Waschechtheit, erzielt.

Der vorliegenden Erfindung lag weiterhin die Aufgabe zu Grunde, Zusammensetzungen und Verfahren zur Färbung keratinhaltiger Fasern, insbesondere menschlicher Haare, unter Verwendung von Naturfarbstoffen bereitzustellen, die eine möglichst geringe Schädigung oder Schwächung der Keratinstruktur hervorrufen.

Eine weitere Aufgabe war es, Zusammensetzungen und Verfahren zur Färbung keratinhaltiger Fasern, insbesondere menschlicher Haare, unter Verwendung von Naturfarbstoffen bereitzustellen, die helle Farbtöne mit hoher Farbintensität oder hoher Chromatizität erzielen.

Überraschend wurde gefunden, dass die in den Patentansprüchen beschriebenen Färbezusammensetzungen und Färbeverfahren eine bequeme Haarfärbung ermöglichen, mit der sich helle Farbtöne mit zufriedenstellender Waschechtheit erzielen lassen, die vergleichbar sind mit einer demi-permanenten Haarfärbung, also einer oxidativen Haarfärbung unter milden Bedingungen (schwach alkalisches Medium, geringe Wasserstoffperoxid-Konzentration), wobei die Haarstruktur aber nicht geschädigt wird.

Ein erster Gegenstand der vorliegenden Erfindung ist eine kosmetische Zusammensetzung zur Oxidationsmittel-freien und Beizmittel-freien Färbung von keratinischen Fasern, insbesondere menschlichen Haaren, mit Naturfarbstoffen, enthaltend:
i. mindestens einen färbenden Extrakt aus einem Farbholz, weiterhin
ii. Wasser,
iii. ein Puffersystem, ausgewählt aus einer Mischung aus einer mittelstarken oder schwachen Säure mit ihrer konjugierten bzw. korrespondierenden Base (bzw. des jeweiligen Salzes) und einer Mischung aus einer mittelstarken oder schwachen Base mit ihrer konjugierten bzw. korrespondierenden Säure,
iv. wobei die Zusammensetzung einen pH-Wert im Bereich von 3,0 - 5,9 aufweist, gemessen bei 20°C,
wobei die Zusammensetzung kein Beizmittel, ausgewählt aus den Salzen von Magnesium, Calcium, Aluminium, von Übergangsmetallen und von Lanthanoiden, kein Cystein, kein N-Acetylcystein, kein Oxidationsfarbstoffvorprodukt und keine Fettsubstanzen, die bei 20°C eine Wasserlöslichkeit von maximal 0,01 g/l aufweisen, enthält.

Alle Löslichkeitsangaben beziehen sich auf einen Normaldruck von 1013 hPa.

Erfindungsgemäße Färbezusammensetzungen enthalten als Naturfarbstoff mindestens einen färbenden Extrakt aus einem Farbholz. Unter Farbhölzern versteht man Holzarten, die einen zum Färben geeigneten Farbstoff enthalten. Dazu zählen das Blauholz (*Haematoxylum campechianum,* Blutholzbaum, Blauholzbaum, Campechebaum), Fisettholz (Fustik, Perückenbaum, *Rhus cotinus, Cotinus coggygria*), Gelbholz *(Maclura tinctoria,* Färbermaulbeerbaum) und Rotholz (*Haematoxylum brasiletto L.,* Brasilianischer Blutholzbaum).

Erfindungsgemäß bevorzugte Färbezusammensetzungen und Färbeverfahren sind dadurch gekennzeichnet, dass der mindestens eine färbende Farbholz-Extrakt aus Farbhölzern stammt, die ausgewählt sind aus Blauholz (*Haematoxylum campechianum,* Blutholzbaum, Blauholzbaum, Campechebaum), Fisettholz (Fustik, Perückenbaum, *Rhus cotinus, Cotinus coggygria*)*,* Gelbholz *(Maclura tinctoria,* Färbermaulbeerbaum) und Rotholz (*Haematoxylum brasiletto L.,* Brasilianischer Blutholzbaum) sowie aus Mischungen dieser Farbhölzer.

Erfindungsgemäß bevorzugte Färbezusammensetzungen und Färbeverfahren sind dadurch gekennzeichnet, dass der mindestens eine färbende Farbholz-Extrakt aus dem Kernholz von Farbhölzern stammt, die ausgewählt sind aus Blauholz (*Haematoxylum campechianum,* Blutholzbaum, Blauholzbaum, Campechebaum), Fisettholz (Fustik, Perückenbaum, *Rhus cotinus, Cotinus coggygria*), Gelbholz *(Maclura tinctoria,* Färbermaulbeerbaum) und Rotholz (*Haematoxylum brasiletto L.,* Brasilianischer Blutholzbaum) sowie aus Mischungen dieser Farbhölzer.

Geeignete Extraktionsmittel sind Wasser, insbesondere heißes Wasser mit einer Temperatur von 45 - 100 °C, weiterhin C₁-C₄-Alkanole und C₂-C₄-Polyole, insbesondere Ethanol, Isopropanol, n-Propanol, Ethylenglycol, 1,2-Propandiol, Glycerin und 1,3-Butylenglycol, sowie Mischungen dieser Extraktionsmittel, insbesondere Mischungen aus Wasser und mindestens einem C₁-C₄-Alkanol, Mischungen aus Wasser und mindestens einem C₂-C₄-Polyol, besonders bevorzugt Wasser/Ethanol-Mischungen. Ein erfindungsgemäß außerordentlich bevorzugtes Extraktionsmittel ist Wasser.

Weitere erfindungsgemäß besonders bevorzugte Färbezusammensetzungen und Färbeverfahren sind dadurch gekennzeichnet, dass ein Extrakt aus dem Kernholz des Perückenbaums (*Rhus cotinus, Cotinus coggygria*) eingesetzt wird, wobei der Extrakt durch Extraktion mit Wasser, bevorzugt mit Wasser einer Temperatur von 45 - 100 °C erhalten wurde. Der Extrakt selbst wird bevorzugt in Pulverform, erhältlich durch Trocknung, bevorzugt Sprühtrocknung, des wässrigen Extrakts, verwendet.

Weitere erfindungsgemäß besonders bevorzugte Färbezusammensetzungen und Färbeverfahren sind dadurch gekennzeichnet, dass ein Extrakt aus dem Kernholz des Brasilianischen Blutholzbaums (*Haematoxylum brasiletto L.*) eingesetzt wird, wobei der Extrakt durch Extraktion mit Wasser, bevorzugt mit Wasser einer Temperatur von 45 - 100 °C, erhalten wurde. Der Extrakt selbst wird bevorzugt in Pulverform, erhältlich durch Trocknung, bevorzugt Sprühtrocknung, des wässrigen Extrakts, verwendet. Andere erfindungsgemäß bevorzugte Extrakte aus dem Kernholz von *Haematoxylum brasiletto L.* werden in konzentrierter Form, erhältlich durch teilweise Abdestillation des Extraktionsmittels nach der Extraktion, als viskose Flüssigkeit eingesetzt.

Weitere erfindungsgemäß besonders bevorzugte Färbezusammensetzungen und Färbeverfahren sind dadurch gekennzeichnet, dass ein Extrakt aus dem Kernholz des Blutholzbaums (*Haematoxylum campechianum,* auch als Blauholzbaum oder Campechebaum bezeichnet) eingesetzt wird, wobei der Extrakt durch Extraktion mit Wasser, bevorzugt mit Wasser einer Temperatur von 45 - 100 °C, erhalten wurde. Der Extrakt selbst wird bevorzugt in konzentrierter Form, erhältlich durch teilweise Abdestillation des Extraktionsmittels nach der Extraktion, als viskose Flüssigkeit eingesetzt. Andere erfindungsgemäß bevorzugte Extrakte aus dem Kernholz von *Haematoxylum campechianum* werden in Pulverform, erhältlich durch Trocknung, bevorzugt Sprühtrocknung, des wässrigen Extrakts, verwendet.

Ein bevorzugtes Polyphenol aus *Haematoxylum campechianum* ist Hämatoxylin.

Eine der primären Farbkomponenten aus *Haematoxylum campechianum* ist das Neoflavonoid Hämatein. Hämatein entsteht durch Fermentation von Hämatoxylin, das aus dem Kernholz von *Haematoxylum campechianum* extrahiert wird. Hämatein (Molmasse 300,26 g/mol) hat die folgende Strukturformel (I):

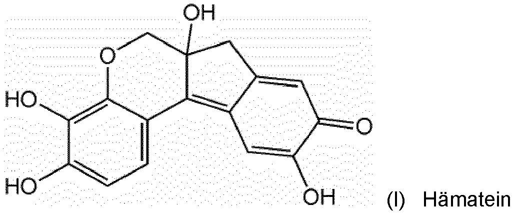

Weitere erfindungsgemäß besonders bevorzugte Färbezusammensetzungen und Färbeverfahren sind dadurch gekennzeichnet, dass das mindestens eine Polyphenol ausgewählt ist aus Hämatein und Hämatoxylin sowie Mischungen hiervon.

Erfindungsgemäß außerordentlich bevorzugte Färbezusammensetzungen und Färbeverfahren sind dadurch gekennzeichnet, dass, wenn das Polyphenol aus Hämatoxylin ausgewählt ist, außer Hämatoxylin und seinem Fermentationsprodukt Hämatein kein weiteres Polyphenol enthalten ist.

Weitere erfindungsgemäß besonders bevorzugte Färbezusammensetzungen und Färbeverfahren sind dadurch gekennzeichnet, dass das mindestens eine Polyphenol, das bevorzugt ausgewählt ist aus Tanninen und Pseudotanninen, in Form eines Extrakts aus dem Kernholz des Färbermaulbeerbaums (*Maclura tinctoria,* auch *Chlorophora tinctoria* oder *Morus tinctoria*) eingesetzt wird, wobei der Extrakt durch Extraktion mit Wasser, bevorzugt mit Wasser einer Temperatur von 45 - 100 °C, erhalten wurde. Der Extrakt selbst wird bevorzugt in konzentrierter Form, erhältlich durch teilweise Abdestillation des Extraktionsmittels nach der Extraktion, als viskose Flüssigkeit eingesetzt. Andere erfindungsgemäß bevorzugte Extrakte aus dem Kernholz von *Haematoxylum brasiletto L.* werden in Pulverform, erhältlich durch Trocknung, bevorzugt Sprühtrocknung, des wässrigen Extrakts, verwendet.

Erfindungsgemäß bevorzugte Färbezusammensetzungen und Färbeverfahren sind dadurch gekennzeichnet, dass die Zusammensetzung, jeweils bezogen auf ihr Gewicht, den mindestens einen färbenden Farbholz-Extrakt i jeweils in einer Menge von 0,1 - 20 Gew.-%, bevorzugt 0,2 - 10 Gew.-%, besonders bevorzugt 0,5 - 5 Gew.-%, außerordentlich bevorzugt 1 - 4 Gew.-%, noch weiter bevorzugt 2 - 3 Gew.-% enthält.

### Polyphenole

Die färbenden Bestandteile, die aus den Farbhölzern extrahiert werden, stellen Polyphenole dar. Gemäß der Definition von Polyphenolen nach Quideau sind Polyphenole Verbindungen, die auf dem Shikimat-Biosyntheseweg erhältlich sind und weiterhin mindestens 2 Hydroxygruppen im Molekül aufweisen und deren Molmasse im Bereich von 170 bis 20.000 g/mol, bevorzugt 290 bis 3000 g/mol, liegt. Erfindungsgemäß bevorzugte Polyphenole weisen mindestens 12 Hydroxygruppen und mindestens fünf Phenylgruppen auf.

Die in erfindungsgemäß bevorzugten Färbezusammensetzungen und Färbeverfahren verwendeten Farbholz-Extrakte weisen üblicherweise, bezogen auf ihr Gewicht, einen Gesamtgehalt an Polyphenolen von 10 - 100 Gew.-%, bevorzugt 15 - 95 Gew.-%, besonders bevorzugt 20 - 80 Gew.-%, außerordentlich bevorzugt 30 - 70 Gew.-%, auf.

Erfindungsgemäß bevorzugte Färbezusammensetzungen und Färbeverfahren sind dadurch gekennzeichnet, dass die Zusammensetzung, jeweils bezogen auf ihr Gewicht, mindestens ein Polyphenol, jeweils in einer Menge von 0,05 - 10 Gew.-%, bevorzugt 0,1 - 5 Gew.-%, besonders bevorzugt 0,5 - 4 Gew.-%, außerordentlich bevorzugt 1 - 2 Gew.-%, enthält.

Erfindungsgemäß bevorzugte Polyphenole sind ausgewählt aus Brasilin, Brasilein, Hämatoxylin, Hämatein, Maclurin, Kämpferol, Morin sowie Mischungen hiervon.

Erfindungsgemäß bevorzugte Färbezusammensetzungen und Färbeverfahren sind dadurch gekennzeichnet, dass der mindestens eine färbende Farbholz-Extrakt i ein Polyphenol, ausgewählt aus Brasilin, Brasilein, Hämatoxylin, Hämatein, Maclurin, Kämpferol, Morin sowie Mischungen hiervon, enthält.

Weitere erfindungsgemäß bevorzugte Färbezusammensetzungen und Färbeverfahren sind dadurch gekennzeichnet, dass die Zusammensetzung, jeweils bezogen auf ihr Gewicht, mindestens ein Polyphenol, ausgewählt aus Brasilin, Brasilein, Hämatoxylin, Hämatein, Maclurin, Kämpferol, Morin sowie Mischungen hiervon, jeweils in einer Menge von 0,05 - 10 Gew.-%, bevorzugt 0,1 - 5 Gew.-%, besonders bevorzugt 0,5 - 4 Gew.-%, außerordentlich bevorzugt 1 - 2 Gew.-%, enthält.

### Wasser

In erfindungsgemäß bevorzugten Färbezusammensetzungen und Färbeverfahren beträgt der Wassergehalt der Färbezusammensetzung 40 - 98 Gew.-%, bevorzugt 50 - 97 Gew.-%, besonders bevorzugt 60 - 85 Gew.-%, jeweils bezogen auf das Gewicht der Färbezusammensetzung.

### pH-Wert

Die erfindungsgemäße Färbezusammensetzung weist einen pH-Wert im Bereich von 3,0 bis 5,9, bevorzugt im Bereich von 4,0 bis 5,5, besonders bevorzugt im Bereich von 4,5 bis 5,2, außerordentlich bevorzugt im Bereich von 4,8 bis 5,0, jeweils gemessen bei 20°C, auf. In diesem pH-Bereich wurden besonders helle, leuchtende Farbtöne im rotblonden bis hellbraunen Bereich erzielt.

Die im erfindungsgemäßen Färbeverfahren eingesetzte Färbezusammensetzung weist einen pH-Wert im Bereich von 3,0 bis 10,5, bevorzugt im Bereich von 4,0 bis 10,0, besonders bevorzugt im Bereich von 5,5 bis 9,5, außerordentlich bevorzugt im Bereich von 7,0 bis 8,0, jeweils gemessen bei 20°C, auf. In sauren pH-Bereich wurden besonders helle, leuchtende Farbtöne im rotblonden bis hellbraunen Bereich erzielt. Im neutralen bis alkalischen Bereich wurden, auch ohne den Zusatz von Beizmitteln, dunklere Farbtöne erzielt.

### Puffer

Die erfindungsgemäße Färbezusammensetzung enthält weiterhin ein Puffersystem, ausgewählt aus einer Mischung aus einer mittelstarken oder schwachen Säure mit ihrer konjugierten bzw. korrespondierenden Base (bzw. des jeweiligen Salzes) und einer Mischung aus einer mittelstarken oder schwachen Base mit ihrer konjugierten bzw. korrespondierenden Säure.

Erfindungsgemäß bevorzugt geeignete korrespondierende Säure-Base-Paare sind solche, die die erfindungsgemäße Färbezusammensetzung im pH-Wert-Bereich von 3,0 bis 5,9, bevorzugt im Bereich von 4,0 bis 5,5, besonders bevorzugt im Bereich von 4,5 bis 5,2, außerordentlich bevorzugt im Bereich von 4,8 bis 5,0, jeweils gemessen bei 20°C, stabilisieren. Erfindungsgemäß besonders bevorzugte Puffersysteme sind ausgewählt aus
- Mischungen aus Citronensäure und ihren Salzen, insbesondere den Alkalimetallcitraten, insbesondere den Natriumsalzen, insbesondere Trinatriumcitrat,
- Mischungen aus Weinsäure und ihren Salzen, insbesondere den Alkalimetalltartraten, insbesondere den Kaliumsalzen, insbesondere Kaliumhydrogentartrat,
- Mischungen aus Phthalsäure und ihren Salzen, insbesondere den Kaliumsalzen, insbesondere Kaliumhydrogenphthalat,
- Mischungen aus Milchsäure und ihren Salzen, insbesondere Milchsäure/Natriumlactat-Mischungen,
- Mischungen aus Gluconsäure und ihren Salzen, insbesondere Gluconsäure/Natriumgluconat-Mischungen,
- Mischungen aus Bernsteinsäure und ihren Salzen, insbesondere den Natriumsalzen, insbesondere Natriumhydrogensuccinat und Dinatriumsuccinat, sowie
- Mischungen aus Äpfelsäure und ihren Salzen, insbesondere den Natriumsalzen, insbesondere Natriumhydrogenmalat und Dinatriummalat.

Ein erfindungsgemäß besonders bevorzugtes Puffersystem ist aus Citronensäure und mindestens einem Natriumsalz der Citronensäure gebildet; außerordentlich bevorzugt sind Mischungen aus Citronensäure und Trinatriumcitrat.

Andere Puffersysteme, z.B. Essigsäure/Natriumacetat, sind prinzipiell ebenfalls erfindungsgemäß geeignet. Aufgrund des Essiggeruchs ist ein solcher Puffer allerdings nicht für die Herstellung eines kosmetischen Marktproduktes akzeptabel.

Erfindungsgemäß bevorzugte Färbezusammensetzungen sind dadurch gekennzeichnet, dass sie, jeweils bezogen auf ihr Gewicht, ein Puffersystem in einer Menge von 0,5 - 5 Gew.-%, bevorzugt 0,8 - 4,5 Gew.-%, besonders bevorzugt 1,5 - 3,5 Gew.-%, außerordentlich bevorzugt 2,1 - 3,0 Gew.-%, enthalten.

Erfindungsgemäß bevorzugte Färbezusammensetzungen sind dadurch gekennzeichnet, dass sie, jeweils bezogen auf ihr Gewicht, als Puffersystem 0,2 - 1,5 Gew.-%, bevorzugt 0,3 - 1,4 Gew.-%, besonders bevorzugt 0,5 - 1,1 Gew.-%, außerordentlich bevorzugt 0,6 - 0,9 Gew.-% Citronensäure und 0,3 - 3,5 Gew.-%, bevorzugt 0,5 - 3,1 Gew.-%, besonders bevorzugt 1,0 - 2,4 Gew.-%, außerordentlich bevorzugt 1,5 - 2,1 Gew.-% Trinatriumcitrat enthalten.

Erfindungsgemäß bevorzugte Färbezusammensetzungen sind dadurch gekennzeichnet, dass sie, jeweils bezogen auf ihr Gewicht, als Puffersystem 0,2 - 1,5 Gew.-%, bevorzugt 0,3 - 1,4 Gew.-%, besonders bevorzugt 0,5 - 1,1 Gew.-%, außerordentlich bevorzugt 0,6 - 0,9 Gew.-% Gluconsäure und 0,3 - 3,5 Gew.-%, bevorzugt 0,5 - 3,1 Gew.-%, besonders bevorzugt 1,0 - 2,4 Gew.-%, außerordentlich bevorzugt 1,5 - 2,1 Gew.-% Natriumgluconat enthalten.

Erfindungsgemäß bevorzugte Färbezusammensetzungen sind dadurch gekennzeichnet, dass sie, jeweils bezogen auf ihr Gewicht, als Puffersystem 0,2 - 1,5 Gew.-%, bevorzugt 0,3 - 1,4 Gew.-%, besonders bevorzugt 0,5 - 1,1 Gew.-%, außerordentlich bevorzugt 0,6 - 0,9 Gew.-% Milchsäure und 0,3 - 3,5 Gew.-%, bevorzugt 0,5 - 3,1 Gew.-%, besonders bevorzugt 1,0 - 2,4 Gew.-%, außerordentlich bevorzugt 1,5 - 2,1 Gew.-% Natriumlactat enthalten.

Erfindungsgemäß bevorzugte Färbezusammensetzungen sind dadurch gekennzeichnet, dass sie, jeweils bezogen auf ihr Gewicht, als Puffersystem 0,2 - 1,5 Gew.-%, bevorzugt 0,3 - 1,4 Gew.-%, besonders bevorzugt 0,5 - 1,1 Gew.-%, außerordentlich bevorzugt 0,6 - 0,9 Gew.-% Bernsteinsäure und 0,3 - 3,5 Gew.-%, bevorzugt 0,5 - 3,1 Gew.-%, besonders bevorzugt 1,0 - 2,4 Gew.-%, außerordentlich bevorzugt 1,5 - 2,1 Gew.-% Dinatriumsuccinat enthalten.

Erfindungsgemäß bevorzugte Färbezusammensetzungen sind dadurch gekennzeichnet, dass sie, jeweils bezogen auf ihr Gewicht, als Puffersystem 0,2 - 1,5 Gew.-%, bevorzugt 0,3 - 1,4 Gew.-%, besonders bevorzugt 0,5 - 1,1 Gew.-%, außerordentlich bevorzugt 0,6 - 0,9 Gew.-% Bernsteinsäure und 0,3 - 3,5 Gew.-%, bevorzugt 0,5 - 3,1 Gew.-%, besonders bevorzugt 1,0 - 2,4 Gew.-%, außerordentlich bevorzugt 1,5 - 2,1 Gew.-% Natriumhydrogensuccinat enthalten.

Erfindungsgemäß bevorzugte Färbezusammensetzungen sind dadurch gekennzeichnet, dass sie, jeweils bezogen auf ihr Gewicht, als Puffersystem 0,2 - 1,5 Gew.-%, bevorzugt 0,3 - 1,4 Gew.-%, besonders bevorzugt 0,5 - 1,1 Gew.-%, außerordentlich bevorzugt 0,6 - 0,9 Gew.-% Äpfelsäure und 0,3 - 3,5 Gew.-%, bevorzugt 0,5 - 3,1 Gew.-%, besonders bevorzugt 1,0 - 2,4 Gew.-%, außerordentlich bevorzugt 1,5 - 2,1 Gew.-% Dinatriummalat enthalten.

Erfindungsgemäß bevorzugte Färbezusammensetzungen sind dadurch gekennzeichnet, dass sie, jeweils bezogen auf ihr Gewicht, als Puffersystem 0,2 - 1,5 Gew.-%, bevorzugt 0,3 - 1,4 Gew.-%, besonders bevorzugt 0,5 - 1,1 Gew.-%, außerordentlich bevorzugt 0,6 - 0,9 Gew.-% Äpfelsäure und 0,3 - 3,5 Gew.-%, bevorzugt 0,5 - 3,1 Gew.-%, besonders bevorzugt 1,0 - 2,4 Gew.-%, außerordentlich bevorzugt 1,5 - 2,1 Gew.-% Natriumhydrogenmalat enthalten.

Auch die im erfindungsgemäßen Färbeverfahren eingesetzte Färbezusammensetzung enthält ein Puffersystem, ausgewählt aus einer Mischung aus einer mittelstarken oder schwachen Säure mit ihrer konjugierten bzw. korrespondierenden Base (bzw. des jeweiligen Salzes) und einer Mischung aus einer mittelstarken oder schwachen Base mit ihrer konjugierten bzw. korrespondierenden Säure. Für das erfindungsgemäße Färbeverfahren bevorzugt geeignete korrespondierende Säure-Base-Paare sind solche, die die erfindungsgemäße Färbezusammensetzung im pH-Wert-Bereich von 3,0 bis 10,5, bevorzugt im Bereich von 4,0 bis 10,0, besonders bevorzugt im Bereich von 5,5 bis 9,5, außerordentlich bevorzugt im Bereich von 7,0 bis 8,0, jeweils gemessen bei 20°C, stabilisieren. Erfindungsgemäß besonders bevorzugte Puffersysteme sind ausgewählt aus
- Mischungen aus Phosphat- und Hydrogenphosphat-Salzen, insbesondere den Alkalimetallsalzen, insbesondere den Natriumsalzen, insbesondere Trinatriumphosphat/Dinatriumhydrogenphosphat-Mischungen und Dinatriumhydrogenphosphat/Natriumdihydrogenphosphat-Mischungen,
- Mischungen aus Carbonat- und Hydrogencarbonat-Salzen, insbesondere den Alkalimetallsalzen, insbesondere den Natriumsalzen, insbesondere Dinatriumcarbonat/Natriumhydrogencarbonat-Mischungen,
- Mischungen aus Citronensäure und ihren Salzen, insbesondere den Alkalimetallcitraten, insbesondere den Natriumsalzen, insbesondere Trinatriumcitrat,
- Mischungen aus Weinsäure und ihren Salzen, insbesondere den Alkalimetalltartraten, insbesondere den Kaliumsalzen, insbesondere Kaliumhydrogentartrat,
- Mischungen aus Phthalsäure und ihren Salzen, insbesondere den Kaliumsalzen, insbesondere Kaliumhydrogenphthalat,
- Mischungen aus Milchsäure und ihren Salzen, insbesondere Milchsäure/Natriumlactat-Mischungen,
- Mischungen aus Gluconsäure und ihren Salzen, insbesondere Gluconsäure/Natriumgluconat-Mischungen,
- Mischungen aus Bernsteinsäure und ihren Salzen, insbesondere den Natriumsalzen, insbesondere Natriumhydrogensuccinat und Dinatriumsuccinat, sowie
- Mischungen aus Äpfelsäure und ihren Salzen, insbesondere den Natriumsalzen, insbesondere Natriumhydrogenmalat und Dinatriummalat.

In erfindungsgemäß bevorzugten Färbezusammensetzungen beträgt der Wassergehalt 40 - 95 Gew.-%, bevorzugt 50 - 90 Gew.-%, besonders bevorzugt 60 - 85 Gew.-%, jeweils bezogen auf das Gewicht der Färbezusammensetzung.

Zur Verbesserung des Färbeergebnisses kann es bevorzugt sein, dass die erfindungsgemäßen Färbezusammensetzungen Natriumsulfit (Na₂SO₃) enthalten. Besonders bevorzugt kann Natriumsulfit (Na₂SO₃) in einer Menge von 0,1 bis 3,0 Gew.-%, bevorzugt 0,5 bis 2 Gew.-%, besonders bevorzugt 0,7 bis 1,5 Gew.-%, außerordentlich bevorzugt 1,0 bis 1,2 Gew.-%, jeweils bezogen auf das Gewicht der Zusammensetzung, enthalten.

Überraschenderweise wurde festgestellt, dass das Reduktionsmittel Natriumdisulfit mit der Formel Na₂S₂O₅, das auch als Natriummetabisulfit bezeichnet wird, die Färbeergebnisse eher verschlechtert. Erfindungsgemäß besonders bevorzugte Färbezusammensetzungen und Färbeverfahren sind daher dadurch gekennzeichnet, dass die Färbezusammensetzung kein Metabisulfit, insbesondere kein Natriummetabisulfit, enthält.

Die erfindungsgemäßen Färbezusammensetzungen sind weiterhin dadurch gekennzeichnet, dass sie kein Cystein und keine Cystein-Derivate, insbesondere kein N-Acetylcystein, enthalten. Unter den Ausdruck "Cystein" fallen auch die Salze des Cysteins, wie beispielsweise das Cysteinhydrochlorid. Unter den Ausdruck "Cystein-Derivate" fallen insbesondere die N-Acylcysteine, insbesondere das N-Acetylcystein. Cystein und Cystein-Derivate sind in der Lage, die Disulfidbrücken im Keratin zu reduzieren. Derartige Reaktionsmechanismen schwächen die Faserstruktur, was erfindungsgemäß unerwünscht ist.

Die erfindungsgemäßen Färbezusammensetzungen und Färbeverfahren kommen ohne den Zusatz von so genannten Beizmitteln aus. Unter Beizmitteln werden im Sinne der vorliegenden Anmeldung Salze von Magnesium, Calcium, Aluminium, von Übergangsmetallen und von Lanthanoiden verstanden. Insbesondere benötigen die erfindungsgemäßen Färbezusammensetzungen und Färbeverfahren nicht den Zusatz eines Eisen(II)-salzes, Eisen(III)-Salzes oder eines Titan-Salzes.

Die erfindungsgemäßen Färbezusammensetzungen und Färbeverfahren kommen ohne den Zusatz von Oxidationsfarbstoffvorprodukten aus. Oxidationsfarbstoffvorprodukte sind fachnotorisch bekannt; vorliegend wird zum Beispiel Bezug genommen auf die in DE102016225379A1 genannten Oxidationsfarbstoffvorprodukte.

Die erfindungsgemäßen Färbezusammensetzungen und Färbeverfahren kommen ohne den Zusatz von Fettsubstanzen, die bei 20°C eine Wasserlöslichkeit von maximal 0,01 g/l oder weniger aufweisen, aus. Es wurde festgestellt, dass wasserunlösliche Fettsubstanzen, das Färbeergebnis beeinträchtigen können. Ohne an diese Theorie gebunden sein zu wollen, wird vermutet, dass eine Hydrophobierung der Keratinfasern erfolgt, die den Farbaufzug behindert.

Beispiele für wasserunlösliche Fettsubstanzen, die in den erfindungsgemäßen Zusammensetzungen und Verfahren nicht enthalten sein sollen, sind pflanzliche Buttern, insbesondere Schibutter (Butyrospermum parkii), Kakaobutter (Theobroma cacao), Butter aus Shorea stenoptera, Illipebutter, Butter aus Madhuca-Spezies, Mangobutter (Mangifera indica), Murumurubutter, Butter aus Garcinia Indica, Butter aus Virola sebifera, tierische Buttern, Pflanzenöle, gehärtete Pflanzenöle, pflanzliche Wachse, z. B. Candelillawachs, Carnaubawachs, Sonnenblumenwachs, Fruchtwachse wie Orangenwachse, tierische Wachse, z. B. Bienenwachs, Schellackwachs und Walrat, hydrierte oder gehärtete Wachse, chemisch modifizierte Wachse, insbesondere die Hartwachse, wie z. B. Montanesterwachse, hydrierte Jojobawachse und Sasolwachse, Polyalkylenwachse und Polyethylenglycolwachse, C₂₀-C₄₀-Dialkylester von Dimersäuren, C₃₀₋₅₀-Alkylbienenwachs, Alkyl- und Alkylarylester von Dimerfettsäuren, so genannte Fettalkohole, also nicht-alkoxylierte Alkanole und Alkenole mit mindestens 8 Kohlenstoffatomen, die linear oder verzweigt, gesättigt oder ungesättigt sein können, die Mono-, Di- und Triester von C2-C6-Polyolen, wie Glycerin oder Ethylenglycol, mit Fettsäuren mit mindestens 8 Kohlenstoffatomen, die linear oder verzweigt, gesättigt oder ungesättigt sein können, weiterhin die nicht-alkoxylierten Fettsäuren mit mindestens 8 Kohlenstoffatomen, die linear oder verzweigt, gesättigt oder ungesättigt sein können.

Tenside und Emulgatoren können fettähnlich aufgebaut sein, fallen aber nicht unter die wasserunlöslichen Fettsubstanzen im Sinne dieser Anmeldung. Tenside und Emulgatoren im Sinne der vorliegenden Anmeldung sind amphiphile (bifunktionelle) Verbindungen, die aus mindestens einem hydrophoben und mindestens einem hydrophilen Molekülteil bestehen. Der hydrophobe Rest ist bevorzugt eine Kohlenwasserstoffkette mit 8-28 Kohlenstoff-Atomen, die gesättigt oder ungesättigt, linear oder verzweigt sein kann. Besonders bevorzugt ist diese C₈-C₂₈-Alkylkette linear. Basiseigenschaften der Tenside und Emulgatoren sind die orientierte Absorption an Grenzflächen in wässrigen Systemen sowie die Aggregation zu Mizellen und die Ausbildung von lyotropen Phasen in wässrigen Systemen. Wasserunlösliche Fettsubstanzen hingegen bilden - für sich genommen - in wässrigen Systemen keine Mizellen.

Duftstoffverbindungen bzw. Riechstoffverbindungen, wie nachstehend definiert, fallen ebenfalls nicht unter die vorstehend definierten wasserunlöslichen Fettsubstanzen.

Ein weiterer Gegenstand der vorliegenden Erfindung ist ein Verfahren zur Oxidationsmittel-freien und Beizmittel-freien Färbung von keratinischen Fasern, insbesondere menschlichen Haaren, mit Naturfarbstoffen, wobei eine Färbezusammensetzung, enthaltend
i. mindestens einen färbenden Extrakt aus einem Farbholz,
ii. Wasser,
iii. ein Puffersystem, ausgewählt aus einer Mischung aus einer mittelstarken oder schwachen Säure mit ihrer konjugierten bzw. korrespondierenden Base (bzw. des jeweiligen Salzes) und einer Mischung aus einer mittelstarken oder schwachen Base mit ihrer konjugierten bzw. korrespondierenden Säure,
iv. wobei die Färbezusammensetzung einen pH-Wert im Bereich von 3,0 - 10,5 aufweist, gemessen bei 20°C,
wobei die Färbezusammensetzung kein Beizmittel, ausgewählt aus den Salzen von Magnesium, Calcium, Aluminium, von Übergangsmetallen und von Lanthanoiden, kein Cystein, kein N-Acetylcystein, kein Oxidationsfarbstoffvorprodukt und keine Fettsubstanzen, die bei 20°C eine Wasserlöslichkeit von maximal 0,01 g/l aufweisen, enthält,
auf die keratinischen Fasern, insbesondere auf menschliche Haare, aufgetragen und nach einer Einwirkzeit von 30 Sekunden bis 60 Minuten, bevorzugt 5 bis 45 Minuten, besonders bevorzugt 15 bis 30 Minuten, mit Wasser ausgespült wird,
wobei das Verfahren keine Applikation eines Beizmittels, ausgewählt aus Salzen von Magnesium, Calcium, Aluminium, von Übergangsmetallen und von Lanthanoiden, umfasst.

Ein erfindungsgemäß bevorzugtes Verfahren zur Oxidationsmittel-freien und Beizmittel-freien Färbung von keratinischen Fasern, insbesondere menschlichen Haaren, wie vorstehend beschrieben, ist dadurch gekennzeichnet, dass die keratinischen Fasern in einem Zeitraum von bis zu 7 Tagen vor der Applikation der erfindungsgemäß oder erfindungsgemäß bevorzugt verwendeten Färbezusammensetzung nicht mit einem Oxidationsmittel und nicht mit einem Keratin reduzierenden Mittel behandelt wurden.

Zu den Oxidationsmitteln, die üblicherweise in der Haarkosmetik zu Einsatz kommen, mit denen aber eben erfindungsgemäß keine Haarbehandlung stattfinden soll, und zwar auch nicht als Vorbehandlung, zählen Wasserstoffperoxid, Persulfate, Perbromate, Percarbonate, Perborate und Percarbamide. Luftsauerstoff stellt im erfindungsgemäßen Kontext kein Oxidationsmittel dar.

Zu den Keratin reduzierenden Verbindungen, die üblicherweise in der Haarkosmetik zum Einsatz kommen, mit denen aber eben erfindungsgemäß keine Haarbehandlung stattfinden soll, und zwar auch nicht als Vorbehandlung, zählen Thioglykolsäure, Thiomilchsäure, Cystein, N-Acetylcystein, Cysteamin und die Salze der vorgenannten Verbindungen, sowie Mischungen dieser Keratin reduzierenden Verbindungen.

Bezüglich weiterer bevorzugter Ausführungsformen des erfindungsgemäßen Färbeverfahrens gilt - mit Ausnahme des pH-Werts - mutatis mutandis das zu den erfindungsgemäßen Zusammensetzungen Gesagte.

Die erfindungsgemäßen und erfindungsgemäß bevorzugten Zusammensetzungen können optional weitere Zusatzstoffe enthalten, um die Anwendungseigenschaften dieser Zusammensetzungen zu optimieren. Bevorzugte Zusatzstoffe sind insbesondere Verdickungsmittel, die dafür sorgen, dass die erfindungsgemäßen und erfindungsgemäß bevorzugten Zusammensetzungen während der Anwendung besser auf dem Haar verbleiben.

Erfindungsgemäß besonders bevorzugte Zusammensetzungen enthalten mindestens einen oder mehrere hydrophile Verdicker, der bevorzugt ausgewählt ist aus Polysacchariden, die chemisch und/oder physikalisch modifiziert sein können. Als hydrophile Verdicker sind Verbindungen aus der Gruppe der Polysaccharide erfindungsgemäß besonders bevorzugt, da die Grundgerüste der Polysaccharide natürlichen Ursprungs und biologisch abbaubar sind. Bevorzugte hydrophile Polysaccharid-Verdickungsmittel sind ausgewählt aus Cellulosen, Celluloseethern von C1-C4-Alkoholen, Celluloseestern, Xanthan Gum, Alginsäuren (sowie ihren entsprechenden physiologisch verträglichen Salzen, den Alginaten), Agar Agar (mit dem in Agar Agar als Hauptbestandteil vorhandenen Polysaccharid Agarose), Stärke-Fraktionen und Stärke-Derivaten wie Amylose, Amylopektin und Dextrinen, Karaya-Gummi, Johannisbrotkernmehl, Gummi Arabicum, Pektinen, Dextranen und Guar Gum sowie Mischungen hiervon.

Erfindungsgemäß bevorzugte Celluloseether von C1-C4-Alkoholen und Celluloseester sind ausgewählt aus Methylcellulosen, Ethylcellulosen, Hydroxyalkylcellulosen (wie beispielsweise Hydroxyethylcellulose), Methylhydroxyalkylcellulosen und Carboxymethylcellulosen (wie beispielsweise solche mit der INCI-Bezeichnung Cellulose Gum) sowie ihre physiologisch verträglichen Salze.

In bevorzugten Ausführungsformen ist im Hinblick auf eine zuverlässige Viskositätseinstellung und rückstandsfreie Anwendung auf Keratinfasern und der Kopfhaut als hydrophiler Verdicker Xanthan Gum enthalten. In weiteren bevorzugten Ausführungsformen ist im Hinblick auf eine zuverlässige Viskositätseinstellung und rückstandsfreie Anwendung auf Keratinfasern und der Kopfhaut als hydrophiler Verdicker Carboxymethylcellulose (vorzugsweise Carboxymethylcellulose mit der INCI-Bezeichnung Cellulose Gum) enthalten. Carboxymethylcellulose kann in einer bevorzugten Ausführungsform als einziger hydrophiler Verdicker enthalten sein. Besonders bevorzugt ist eine Kombination von Carboxymethylcellulose und Hydroxyethylcellulose. Auch eine Kombination von Carboxymethylcellulose und Xanthan (vorzugsweise Xanthan mit der INCI-Bezeichnung Xanthan Gum) kann erfindungsgemäß bevorzugt sein.

Erfindungsgemäß besonders bevorzugte Zusammensetzungen enthalten mindestens einen hydrophilen Verdicker in einer Gesamtmenge von 0,1 bis 5 Gew.-%, bevorzugt von 0,5 bis 4 Gew.-%, weiter bevorzugt von 1 bis 3,5 Gew.-% und ganz besonders bevorzugt von 1,2 bis 2 Gew.-%, jeweils bezogen auf das Gewicht der Zusammensetzung.

In einer weiteren bevorzugten Ausführungsform der vorliegenden Erfindung enthalten die erfindungsgemäßen Zusammensetzungen, jeweils bezogen auf ihr Gewicht, 0,1 bis 3 Gew.-%, bevorzugt 0,5 bis 2,5 Gew.-%, weiter bevorzugt 1,2 bis 2,0 Gew.-%, Xanthan Gum.

In einer weiteren bevorzugten Ausführungsform der vorliegenden Erfindung enthalten die erfindungsgemäßen Zusammensetzungen, jeweils bezogen auf ihr Gewicht, 0,1 bis 4 Gew.-%, bevorzugt 1 bis 2,8 Gew.-%, Carboxymethylcellulose.

In einer weiteren bevorzugten Ausführungsform der vorliegenden Erfindung enthalten die erfindungsgemäßen Zusammensetzungen, jeweils bezogen auf ihr Gewicht, 0,1 bis 3 Gew.-%, bevorzugt 0,5 bis 2,5 Gew.-%, weiter bevorzugt 1,2 bis 2,0 Gew.-%, Hydroxyethylcellulose.

Erfindungsgemäß besonders bevorzugte Zusammensetzungen enthalten mindestens ein organisches Lösemittel, das eine Phenylgruppe im Molekül aufweist. Bevorzugt ist dieses Lösemittel ausgewählt aus Phenoxyethanol, Benzylalkohol sowie Mischungen hiervon. Überraschend wurde festgestellt, dass sich derartige aromatische Lösemittel positiv auf die Färbeergebnisse der erfindungsgemäßen Färbezusammensetzungen und des erfindungsgemäßen Färbeverfahrens auswirken können.

In einer weiteren bevorzugten Ausführungsform der vorliegenden Erfindung enthalten die erfindungsgemäßen Zusammensetzungen, jeweils bezogen auf ihr Gewicht, 0,1 bis 3 Gew.-%, bevorzugt 0,5 bis 2,5 Gew.-%, weiter bevorzugt 0,8 bis 1,0 Gew.-%, mindestens eines organischen Lösemittels, das eine Phenylgruppe im Molekül aufweist. In einer weiteren bevorzugten Ausführungsform der vorliegenden Erfindung enthalten die erfindungsgemäßen Färbezusammensetzungen, jeweils bezogen auf ihr Gewicht, 0,1 bis 3 Gew.-%, bevorzugt 0,5 bis 2,5 Gew.-%, weiter bevorzugt 0,8 bis 1,0 Gew.-%, mindestens eines organischen Lösemittels, ausgewählt aus Phenoxyethanol, Benzylalkohol sowie Mischungen hiervon.

Erfindungsgemäß besonders bevorzugte Färbezusammensetzungen sind dadurch gekennzeichnet, dass sie mindestens ein aliphatisches Lösemittel, ausgewählt aus C₁-C₄-Alkanolen und C₂-C₄-Polyolen, enthalten, insbesondere ausgewählt aus Ethanol, Isopropanol, n-Propanol, Ethylenglycol, 1,2-Propandiol, Glycerin und 1,3-Butylenglycol, sowie Mischungen dieser Lösemittel, allerdings nur in einer Gesamtmenge von 0,01 - 8 Gew.-%, bevorzugt 0,1 - 6 Gew.-%, besonders bevorzugt 0,5 - 4 Gew.-%, jeweils bezogen auf das Gewicht der Zusammensetzung.

Andere erfindungsgemäß besonders bevorzugte Färbezusammensetzungen sind dadurch gekennzeichnet, dass sie kein aliphatisches Lösemittel, ausgewählt aus C₁-C₄-Alkanolen und C₂-C₄-Polyolen, enthalten.

Um die erfindungsgemäßen Zusammensetzungen für den Anwender auch sensorisch attraktiv zu gestalten, sind weitere erfindungsgemäß besonders bevorzugte Färbezusammensetzungen dadurch gekennzeichnet, dass sie mindestens ein Parfümöl enthalten, das mindestens eine Duftstoffverbindung bzw. Riechstoffverbindung enthält.

Die Definition eines Riechstoffs im Sinne der vorliegenden Anmeldung entspricht der fachmännisch üblichen Definition, wie sie dem RÖMPP Chemie Lexikon, Stand Dezember 2007, entnommen werden kann. Danach ist ein Riechstoff eine chemische Verbindung mit Geruch und/oder Geschmack, der die Rezeptoren der Haarzellen des olfaktorischen Systems erregt (adäquater Reiz). Die hierzu notwendigen physikalischen und chemischen Eigenschaften sind eine niedrige Molmasse von maximal 300 g/mol, ein hoher Dampfdruck, minimale Wasser- und hohe Lipidlöslichkeit sowie schwache Polarität und das Vorliegen mindestens einer osmophoren Gruppe im Molekül. Um flüchtige, niedermolekulare Substanzen, die üblicherweise und auch im Sinne der vorliegenden Anmeldung nicht als Riechstoff, sondern vornehmlich als Lösemittel angesehen und verwendet werden, wie beispielsweise Ethanol, Propanol, Isopropanol und Aceton, von erfindungsgemäßen Riechstoffen abzugrenzen, weisen erfindungsgemäße Riechstoffe eine Molmasse von 74 bis 300 g/mol auf, enthalten mindestens eine osmophore Gruppe im Molekül und weisen einen Geruch und/oder Geschmack auf, das heißt, sie erregen die Rezeptoren der Haarzellen des olfaktorischen Systems. Beispiele für Duft- und Riechstoffverbindungen vom Typ der Ester sind Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmecyclat. Beispiele für Duft- und Riechstoffverbindungen vom Typ der Ether sind Benzylethylether und Ambroxan, Beispiele für Duft- und Riechstoffverbindungen vom Typ der Aldehyde sind die linearen Alkanale mit 8 - 18 C-Atomen, Citral, Citronellal, Citronellyloxy-acetaldehyd, Cyclamenaldehyd, Lilial und Bourgeonal, Beispiele für Duft- und Riechstoffverbindungen vom Typ der Ketone sind die Jonone, alpha-Isomethylionon und Methylcedrylketon, Beispiele für Duft- und Riechstoffverbindungen vom Typ der Alkohole sind Anethol, Citronellol, Eugenol, Geraniol, Linalool, Phenylethylalkohol und Terpineol, Beispiele für Duft- und Riechstoffverbindungen vom Typ der Terpene sind Limonen und Pinen. Beispiele für Duft- und Riechstoffverbindungen sind Pine-, Citrus-, Jasmin-, Patchouly-, Rosen-, Ylang-Ylang-Öl, Muskateller-Salbeiöl, Kamillenöl, Nelkenöl, Minzöl, Zimtblätteröl, Lindenblütenöl, Wacholderbeeröl, Vetiveröl, Olibanumöl, Galbanumöl, Labdanumöl, Orangenblütenöl, Neroliöl, Orangenschalenöl, weiterhin die ätherischen Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennadelöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Niaouliöl, Orangenöl, Origanum-öl, Palmarosaöl, Patschuliöl, Perubalsamöl, Petitgrainöl, Pfefferöl, Pfefferminzöl, Pimentöl, Pine-Öl, Rosenöl, Rosmarinöl, Sellerieöl, Spiköl, Sternanisöl, Terpentinöl, Thujaöl, Thymianöl, Verbenaöl, Wacholderbeeröl, Wermutöl, Wintergrünöl, Ysop-Öl, Zimtöl, Zitronellöl, Zitronenöl und Zypressenöl. Weitere Duft- und Riechstoffverbindungen sind Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphtholmethylether, Nerol, Nitrobenzol, n-Nonylaldehyd, Nonylakohol, n-Octylaldehyd, p-Oxy-Acetophenon, Pentadecanolid, β-Phenylethylakohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, gamma-Undecalacton, Vanillin, Veratrumaldehyd, Zimtaldehyd, Zimtalkohol, Zimtsäure, Zimtsäureethylester und Zimtsäurebenzylester.

Weitere (leichter flüchtige) Riechstoffe sind Alkylisothiocyanate (Alkylsenföle), Butandion, Limonen, Linalool, Linaylacetat und -propionat, Menthol, Menthon, Methyl-n-heptenon, Phellandren, Phenylacetaldehyd, Terpinylacetat, Zitral und Zitronellal.

Bevorzugt werden Mischungen verschiedener Duftstoffe verwendet, die gemeinsam eine ansprechende Duftnote erzeugen.

Geeignete Parfümöle können auch natürliche Riechstoffgemische enthalten, wie sie aus pflanzlichen oder tierischen Quellen zugänglich sind, z.B. Pinien-, Citrus-, Jasmin-, Rosen-, Lilien- oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Melissenöl, Minzenöl, Zimtblätteröl, Lindenblütenöl, Wacholderbeerenöl, Vetiveröl, Olibanöl, Galbanumöl, Laudanumöl, Gewürznelkenöl, iso-Eugenol, Thymianöl, Bergamotteöl, Geraniumöl und Rosenöl. Erfindungsgemäß außerordentlich bevorzugte Zusammensetzungen sind dadurch gekennzeichnet, dass sie mindestens einen Duftstoff in einer Gesamtmenge von 0,01 - 5 Gew.-%, bevorzugt 0,1 - 3 Gew.-%, besonders bevorzugt 0,5 - 2 Gew.-%, außerordentlich bevorzugt 1 - 1,5 Gew.-%, jeweils bezogen auf das Gewicht der Zusammensetzung, enthalten.

Duftstoffverbindungen bzw. Riechstoffverbindungen, wie vorstehend definiert, fallen nicht unter die vorstehend definierten wasserunlöslichen Fettsubstanzen.

### Ausführungsbeispiel

Das nachstehend dargestellte Ausführungsbeispiel soll den Gegenstand der Erfindung näher erläutern, ohne ihn hierauf zu beschränken.
erfindungsgemäße Färbezusammensetzung (Mengenangaben in Gew.-%)

| | |
|---|---|
| Farbholz-Extrakt * | 2,00 |
| Citrat-Puffer (Natriumsalz) | ad pH 3,0 - 5,9 |
| Wasser, entmineralisiert | ad 100 |
| Gesamt | 100,00 |
| | |
| pH-Wert (20°C) | 3,0 - 5,9 |

Im erfindungsgemäßen Färbeverfahren verwendete Färbezusammensetzung (Mengenangaben in Gew.-%)

| | |
|---|---|
| Farbholz-Extrakt * | 2,00 |
| Phosphat-Puffer (Natriumsalz) | ad pH 7,0 - 8,0 |
| Wasser, entmineralisiert | ad 100 |
| Gesamt | 100,00 |
| | |
| pH-Wert (20°C) | 7,0 - 8,0 |

Im erfindungsgemäßen Färbeverfahren verwendete Färbezusammensetzung (Mengenangaben in Gew.-%)

| | |
|---|---|
| Farbholz-Extrakt * | 2,00 |
| Carbonat-Puffer (Natriumsalz) | ad pH 9,5 - 10,5 |
| Wasser, entmineralisiert | ad 100 |
| Gesamt | 100,00 |
| | |
| pH-Wert (20°C) | 9,5 - 10,5 |

| | |
|---|---|
| * Als Farbholz-Extrakt wurden eingesetzt: * "Otto Dille" Blauholz-Extrakt (*Haematoxylum campechianum*); pulverförmiger Heißwasser-Extrakt aus dem Kernholz von *Haematoxylum campechianum,* pH-Wert der 10 Gew.-%igen Lösung in Wasser: 5,5 ± 1,0 "Otto Dille" Rotholz-Extrakt (*Haematoxylum brasiletto*); pulverförmiger Heißwasser-Extrakt aus dem Kernholz von *Haematoxylum brasiletto,* pH-Wert der 10 Gew.-%igen Lösung in Wasser: 6,5 ± 1,0 "Otto Dille" Gelbholz-Extrakt aus Färbermaulbeerbaum (*Chlorophora tinctoria, Morus tinctoria, Maclura tinctoria*); pulverförmiger Heißwasser-Extrakt aus dem Kernholz von *Maclura tinctoria,* pH-Wert der 10 Gew.-%igen Lösung in Wasser: 5,4 ± 1,0 Bezugsquelle: Baeck GmbH & Co. KG, DE-22844 Norderstedt | |

Die Ausfärbungen wurden auf Strähnen vom Typ vollständig unpigmentiertes kaukasisches Haar der Firma Kerling International Haarfabrik GmbH (Backnang, Deutschland) durchgeführt. Die Strähnen waren 10 cm lang, wovon 8 cm für die Färbung zur Verfügung standen. Die übrigen 2 cm am oberen Ende der Strähnen dienen der Befestigung. Jede Strähne wog 0,45±0,05 Gramm.

Die Strähnen wurden vor dem Färben sauer (pH 4,5) vorgereinigt und getrocknet.

Die Haarsträhnen wurden 30 Minuten lang in der erfindungsgemäßen bzw. erfindungsgemäß verwendeten Färbezusammensetzung gemäß der vorstehenden Tabelle (100 ml / g Strähne) behandelt und dann eine Minute lang unter fließendem entionisiertem Wasser unter 20-maligem Kämmen gespült.

Danach wurden die Haarsträhnen mit einem handelsüblichen Haartrockner in einem definierten Abstand (d = 10 cm) und einer definierten Temperatur (T = 80 ± 5 °C) unter 20-fachem Kämmen mit einem Föhn getrocknet.

Alle farbmetrischen Messungen, nämlich die Bestimmung der Parameter L, a, b im L,a,b-Farbraum, wurden mit dem Farbmetrik-Gerät Spectraflash SF 600 von der Firma Datacolor durchgeführt.

L-Werte beschreiben die schwarz/weiß Skala. Je niedriger der L-Wert ist, desto dunkler ist die Farbe, je höher der L-Wert ist, desto heller ist die Farbe.

a-Werte beschreiben die rot/grün Skala. a-Werte im positiven Bereich repräsentieren rote Farbgebung; a-Werte im negativen Bereich repräsentieren eine grünliche Färbung.

b-Werte beschreiben die gelb/blau Skala. b-Werte im positiven Bereich repräsentieren gelbe Farbgebung; negativer Bereich bläuliche Färbung

### Ergebnis der farbmetrischen Messung für Färbungen mit Blauholz-Extrakt (Mittelwert aus n=10 Messungen)

| | L | a | b |
|---|---|---|---|
| unbehandelte Strähnen | 77,03 | 0,83 | 2,91 |
| Färbung bei pH 3,0 | 67,71 | 10,61 | 32,84 |
| Färbung bei pH 5,0 | 51,53 | 9,77 | 23,78 |
| Färbung bei pH 5,9 | 44,91 | 11,76 | 18,84 |
| Färbung bei pH 7,0 | 42,19 | 8,68 | 8,77 |
| Färbung bei pH 8,0 | 44,02 | 9,54 | 5,19 |
| Färbung bei pH 9,5 | 43,66 | 9,54 | -1,37 |
| Färbung bei pH 10,5 | 44,16 | 6,39 | -6,06 |

Mit steigendem pH nimmt der L-Wert ab, das heißt, die Strähnen werden dunkler. Färbungen mit dem höchsten Rotanteil werden bei pH 3,0 bis 5,9 erzielt.

Es wurde eine konstante Zunahme der Blaufärbung mit steigendem pH beobachtet.

### Waschechtheit der Färbung

Die gefärbten Strähnen wurden 4 Waschzyklen (à 6 Haarwäschen) mit pH 4,5 Shampoo (Schauma 7 Kräuter) einem pH 10 Shampoo unterworfen und farbmetrisch vermessen.

Die Versuche zur Waschechtheit der Färbung zeigten, dass, wenn sich der pH der Färbung und der pH des Shampoos voneinander unterschieden, der Farbstoff ausgewaschen wurde und sich auch der Farbton verschob. Wenn Färbung und Shampoo denselben bzw. ähnlichen pH-Wert aufwiesen, verblasste lediglich die Farbe, während der Farbton erhalten blieb.

## Patentansprüche

1. Kosmetische Zusammensetzung zur Oxidationsmittel-freien und Beizmittel-freien Färbung von keratinischen Fasern, insbesondere menschlichen Haaren, mit Naturfarbstoffen, enthaltend:
i. mindestens einen färbenden Extrakt aus einem Farbholz,
ii. Wasser,
iii. ein Puffersystem, ausgewählt aus einer Mischung aus einer mittelstarken oder schwachen Säure mit ihrer konjugierten bzw. korrespondierenden Base (bzw. des jeweiligen Salzes) und einer Mischung aus einer mittelstarken oder schwachen Base mit ihrer konjugierten bzw. korrespondierenden Säure,
iv. wobei die Zusammensetzung einen pH-Wert im Bereich von 3 - 5,9 aufweist, gemessen bei 20°C,
wobei die Zusammensetzung kein Beizmittel, ausgewählt aus den Salzen von Magnesium, Calcium, Aluminium, von Übergangsmetallen und von Lanthanoiden, kein Cystein, kein N-Acetylcystein, kein Oxidationsfarbstoffvorprodukt und keine Fettsubstanzen, die bei 20°C eine Wasserlöslichkeit von maximal 0,01 g/l aufweisen, enthält.

2. Verfahren zur Oxidationsmittel-freien und Beizmittel-freien Färbung von keratinischen Fasern, insbesondere menschlichen Haaren, mit Naturfarbstoffen, wobei eine Färbezusammensetzung, enthaltend:
i. mindestens einen färbenden Extrakt aus einem Farbholz,
ii. Wasser,
iii. ein Puffersystem, ausgewählt aus einer Mischung aus einer mittelstarken oder schwachen Säure mit ihrer konjugierten bzw. korrespondierenden Base (bzw. des jeweiligen Salzes) und einer Mischung aus einer mittelstarken oder schwachen Base mit ihrer konjugierten bzw. korrespondierenden Säure,
iv. wobei die Färbezusammensetzung einen pH-Wert im Bereich von 3,0 - 10,5 aufweist, gemessen bei 20°C,
wobei die Färbezusammensetzung kein Beizmittel, ausgewählt aus den Salzen von Magnesium, Calcium, Aluminium, von Übergangsmetallen und von Lanthanoiden, kein Cystein, kein N-Acetylcystein, kein Oxidationsfarbstoffvorprodukt und keine Fettsubstanzen, die bei 20°C eine Wasserlöslichkeit von maximal 0,01 g/l aufweisen, enthält,
auf die keratinischen Fasern, insbesondere auf menschliche Haare, aufgetragen und nach einer Einwirkzeit von 30 Sekunden bis 60 Minuten, bevorzugt 5 bis 45 Minuten, besonders bevorzugt 15 bis 30 Minuten, mit Wasser ausgespült wird, wobei das Verfahren keine Applikation eines Beizmittels, ausgewählt aus Salzen von Magnesium, Calcium, Aluminium, von Übergangsmetallen und von Lanthanoiden, umfasst.

3. Zusammensetzung nach Anspruch 1 oder Verfahren nach Anspruch 2, **dadurch gekennzeichnet, dass** der mindestens eine färbende Farbholz-Extrakt i. ausgewählt ist aus Extrakten aus dem Holz von *Haematoxylum brasiletto L.* (Brasilianischer Blutholzbaum), *Haematoxylum campechianum* (Blutholzbaum, Blauholzbaum, Campechebaum), und *Maclura tinctoria* (Färbermaulbeerbaum).

4. Zusammensetzung oder Verfahren nach einem der vorhergehenden Ansprüche, **dadurch gekennzeichnet, dass** der mindestens eine färbende Farbholz-Extrakt i ein Polyphenol, ausgewählt aus Brasilin, Brasilein, Hämatoxylin, Hämatein, Maclurin, Kämpferol, Morin sowie Mischungen hiervon, enthält.

5. Zusammensetzung oder Verfahren nach einem der vorhergehenden Ansprüche, **dadurch gekennzeichnet, dass** die Zusammensetzung, jeweils bezogen auf ihr Gewicht, den mindestens einen färbenden Farbholz-Extrakt i jeweils in einer Menge von 0,1 - 20 Gew.-%, bevorzugt 0,2 - 10 Gew.-%, besonders bevorzugt 0,5 - 5 Gew.-%, außerordentlich bevorzugt 1 - 4 Gew.-%, noch weiter bevorzugt 2 - 3 Gew.-%, enthält.

6. Zusammensetzung oder Verfahren nach einem der vorhergehenden Ansprüche, **dadurch gekennzeichnet, dass** die Zusammensetzung, jeweils bezogen auf ihr Gewicht, mindestens ein Polyphenol, jeweils in einer Menge von 0,05 - 10 Gew.-%, bevorzugt 0,1 - 5 Gew.-%, besonders bevorzugt 0,5 - 4 Gew.-%, außerordentlich bevorzugt 1 - 2 Gew.-%, enthält.

7. Zusammensetzung oder Verfahren nach einem der vorhergehenden Ansprüche, **dadurch gekennzeichnet, dass** die Zusammensetzung, jeweils bezogen auf ihr Gewicht, mindestens ein Polyphenol, ausgewählt aus Brasilin, Brasilein, Hämatoxylin, Hämatein, Maclurin, Kämpferol, Morin sowie Mischungen hiervon, jeweils in einer Menge von 0,05 - 10 Gew.-%, bevorzugt 0,1 - 5 Gew.-%, besonders bevorzugt 0,5 - 4 Gew.-%, außerordentlich bevorzugt 1 - 2 Gew.-%, enthält.

8. Zusammensetzung oder Verfahren nach einem der vorhergehenden Ansprüche, **dadurch gekennzeichnet, dass**, wenn das Polyphenol aus Hämatoxylin ausgewählt ist, außer Hämatoxylin und seinem Fermentationsprodukt Hämatein kein weiteres Polyphenol enthalten ist.

9. Zusammensetzung oder Verfahren nach einem der vorhergehenden Ansprüche, **gekennzeichnet durch** einen Wassergehalt von 40 - 98 Gew.-%, bevorzugt 50 - 97 Gew.-%, besonders bevorzugt 60 - 85 Gew.-%, jeweils bezogen auf ihr Gewicht.

10. Zusammensetzung oder Verfahren nach einem der vorhergehenden Ansprüche, **dadurch gekennzeichnet, dass** kein Metabisulfit enthalten ist.

11. Zusammensetzung nach einem der vorhergehenden Ansprüche, **dadurch gekennzeichnet, dass** die Zusammensetzung einen pH-Wert im Bereich von 4 bis 5,5, bevorzugt im Bereich von 4,5 bis 5,2 besonders bevorzugt im Bereich von 4,8 bis 5,0, jeweils gemessen bei 20°C, aufweist.

12. Zusammensetzung nach einem der vorhergehenden Ansprüche, **dadurch gekennzeichnet, dass** das Puffersystem ausgewählt ist aus
- Mischungen aus Citronensäure und ihren Salzen, insbesondere den Alkalimetallcitraten, insbesondere den Natriumsalzen, insbesondere Trinatriumcitrat,
- Mischungen aus Weinsäure und ihren Salzen, insbesondere den Alkalimetalltartraten, insbesondere den Kaliumsalzen, insbesondere Kaliumhydrogentartrat,
- Mischungen aus Phthalsäure und ihren Salzen, insbesondere den Kaliumsalzen, insbesondere Kaliumhydrogenphthalat,
- Mischungen aus Milchsäure und ihren Salzen, insbesondere Milchsäure/Natriumlactat-Mischungen,
- Mischungen aus Gluconsäure und ihren Salzen, insbesondere Gluconsäure/Natriumgluconat-Mischungen,
- Mischungen aus Bernsteinsäure und ihren Salzen, insbesondere den Natriumsalzen, insbesondere Natriumhydrogensuccinat und Dinatriumsuccinat, sowie
- Mischungen aus Äpfelsäure und ihren Salzen, insbesondere den Natriumsalzen, insbesondere Natriumhydrogenmalat und Dinatriummalat.

13. Verfahren nach einem der vorhergehenden Ansprüche, **dadurch gekennzeichnet, dass** die Färbezusammensetzung einen pH-Wert im Bereich von 4,0 bis 10,0, bevorzugt im Bereich von 5,5 bis 9,5, besonders bevorzugt im Bereich von 7,0 bis 8,0, jeweils gemessen bei 20°C, aufweist.

14. Verfahren nach einem der vorhergehenden Ansprüche, **dadurch gekennzeichnet, dass** das Puffersystem ausgewählt ist aus
- Mischungen aus Phosphat- und Hydrogenphosphat-Salzen, insbesondere den Alkalimetallsalzen, insbesondere den Natriumsalzen, insbesondere Trinatriumphosphat/Dinatriumhydrogenphosphat-Mischungen und Dinatriumhydrogenphosphat/Natriumdihydrogenphosphat-Mischungen,
- Mischungen aus Carbonat- und Hydrogencarbonat-Salzen, insbesondere den Alkalimetallsalzen, insbesondere den Natriumsalzen, insbesondere Dinatriumcarbonat/Natriumhydrogencarbonat-Mischungen,
- Mischungen aus Citronensäure und ihren Salzen, insbesondere den Alkalimetallcitraten, insbesondere den Natriumsalzen, insbesondere Trinatriumcitrat,
- Mischungen aus Weinsäure und ihren Salzen, insbesondere den Alkalimetalltartraten, insbesondere den Kaliumsalzen, insbesondere Kaliumhydrogentartrat,
- Mischungen aus Phthalsäure und ihren Salzen, insbesondere den Kaliumsalzen, insbesondere Kaliumhydrogenphthalat,
- Mischungen aus Milchsäure und ihren Salzen, insbesondere Milchsäure/Natriumlactat-Mischungen,
- Mischungen aus Gluconsäure und ihren Salzen, insbesondere Gluconsäure/Natriumgluconat-Mischungen,
- Mischungen aus Bernsteinsäure und ihren Salzen, insbesondere den Natriumsalzen, insbesondere Natriumhydrogensuccinat und Dinatriumsuccinat, sowie
- Mischungen aus Äpfelsäure und ihren Salzen, insbesondere den Natriumsalzen, insbesondere Natriumhydrogenmalat und Dinatriummalat,

15. Verfahren zur nicht-oxidativen Färbung von keratinischen Fasern, insbesondere menschlichen Haaren, nach einem der vorhergehenden Ansprüche, **dadurch gekennzeichnet, dass** die keratinischen Fasern in einem Zeitraum von bis zu 7 Tagen vor der Applikation der Färbezusammensetzung nicht mit einem Oxidationsmittel und nicht mit einem Keratin reduzierenden Mittel behandelt wurden.

## Claims

1. Cosmetic composition for the oxidant-free and mordant-free dyeing of keratinous fibers, in particular human hair, with natural dyes, containing:
i. at least one coloring extract from a dye wood,
ii. Water,
iii. a buffer system selected from a mixture of a moderately strong or weak acid with its conjugate or corresponding base (or the respective salt) and a mixture of a moderately strong or weak base with its conjugate or corresponding acid,
iv. wherein the composition has a pH value in the range of 3 - 5.9, measured at 20°C,
wherein the composition contains no mordant selected from the salts magnesium, calcium, aluminum, transition metals and lanthanides, no cysteine, no N-acetylcysteine, no oxidation dye precursor and no fatty substances which have a water solubility of at most 0.01 g/l at 20°C.

2. A process for the oxidant-free and mordant-free dyeing of keratinous fibers, in particular human hair, with natural dyes, wherein a dyeing composition comprising:
i. at least one coloring extract from a dye wood,
ii. Water,
iii. a buffer system selected from a mixture of a moderately strong or weak acid with its conjugate or corresponding base (or the respective salt) and a mixture of a moderately strong or weak base with its conjugate or corresponding acid,
iv. wherein the coloring composition has a pH in the range of 3.0 - 10.5, measured at 20°C,
wherein the dyeing composition contains no mordant selected from the salts magnesium, calcium, aluminum, transition metals and lanthanides, no cysteine, no N-acetylcysteine, no oxidation dye precursor and no fatty substances which have a water solubility of at most 0.01 g/l at 20°C,
is applied to the keratinous fibers, in particular to human hair, and rinsed with water after an exposure time of 30 seconds to 60 minutes, preferably 5 to 45 minutes, particularly preferably 15 to 30 minutes, wherein the process does not comprise application of a mordant selected from salts magnesium, calcium, aluminum, transition metals and lanthanides.

3. Composition according to claim 1 or process according to claim 2, **characterized in that** the at least one colouring dye wood extract i. is selected from extracts from the wood of *Haematoxylum brasiletto L.* (Brazilian bloodwood tree), *Haematoxylum campechianum* (bloodwood tree, bluewood tree, campeche tree), and *Maclura tinctoria* (dyer's mulberry tree).

4. Composition or process according to any one of the preceding claims, **characterized in that** the at least one coloring dyewood extract i comprises a polyphenol selected from brazilin, brazilin, hematoxylin, hematein, maclurin, kaempferol, morin and mixtures thereof.

5. Composition or process according to one of the preceding claims, **characterized in that** the composition contains, in each case based on its weight, the at least one colouring coloured wood extract i in an amount of 0.1 - 20 wt.%, preferably 0.2 - 10 wt.%, particularly preferably 0.5 - 5 wt.%, exceptionally preferably 1 - 4 wt.%, still more preferably 2 - 3 wt.%.

6. Composition or process according to one of the preceding claims, **characterized in that** the composition contains, in each case based on its weight, at least one polyphenol, in each case in an amount of 0.05 - 10 wt.%, preferably 0.1 - 5 wt.%, particularly preferably 0.5 - 4 wt.%, exceptionally preferably 1 - 2 wt.%.

7. Composition or process according to one of the preceding claims, **characterized in that** the composition contains, in each case based on its weight, at least one polyphenol selected from brazilin, brazilin, hematoxylin, hematein, maclurin, kaempferol, morin and mixtures thereof, in each case in an amount of 0.05 - 10 wt.%, preferably 0.1 - 5 wt.%, particularly preferably 0.5 - 4 wt.%, exceptionally preferably 1 - 2 wt.%.

8. Composition or process according to any one of the preceding claims, **characterized in that**, when the polyphenol is selected from hematoxylin, no other polyphenol is present except hematoxylin and its fermentation product hematein.

9. Composition or process according to one of the preceding claims, **characterized by** a water content of 40 - 98 wt.%, preferably 50 - 97 wt.%, particularly preferably 60 - 85 wt.%, in each case based on their weight.

10. Composition or process according to one of the preceding claims, **characterized in that** no metabisulphite is present.

11. Composition according to one of the preceding claims, **characterized in that** the composition has a pH value in the range from 4 to 5.5, preferably in the range from 4.5 to 5.2, particularly preferably in the range from 4.8 to 5.0, in each case measured at 20°C.

12. Composition according to any one of the preceding claims, **characterized in that** the buffer system is selected from
- Mixtures of citric acid and its salts, in particular the alkali metal citrates, in particular the sodium salts, in particular trisodium citrate,
- Mixtures of tartaric acid and its salts, in particular the alkali metal tartrates, in particular the potassium salts, in particular potassium hydrogen tartrate,
- Mixtures of phthalic acid and its salts, in particular the potassium salts, especially potassium hydrogen phthalate,
- Mixtures of lactic acid and its salts, in particular lactic acid/sodium lactate mixtures,
- Mixtures of gluconic acid and its salts, in particular gluconic acid/sodium gluconate mixtures,
- Mixtures of succinic acid and its salts, in particular the sodium salts, especially sodium hydrogen succinate and disodium succinate, and
- Mixtures of malic acid and its salts, in particular the sodium salts, especially sodium hydrogen malate and disodium malate.

13. Process according to one of the preceding claims, **characterized in that** the dyeing composition has a pH value in the range from 4.0 to 10.0, preferably in the range from 5.5 to 9.5, particularly preferably in the range from 7.0 to 8.0, in each case measured at 20°C.

14. Method according to one of the preceding claims, **characterized in that** the buffer system is selected from
- Mixtures of phosphate and hydrogen phosphate salts, in particular the alkali metal salts, in particular the sodium salts, in particular trisodium phosphate/disodium hydrogen phosphate mixtures and disodium hydrogen phosphate/sodium dihydrogen phosphate mixtures,
- Mixtures of carbonate and hydrogen carbonate salts, in particular the alkali metal salts, in particular the sodium salts, in particular disodium carbonate/sodium hydrogen carbonate mixtures,
- Mixtures of citric acid and its salts, in particular the alkali metal citrates, in particular the sodium salts, in particular trisodium citrate,
- Mixtures of tartaric acid and its salts, in particular the alkali metal tartrates, in particular the potassium salts, in particular potassium hydrogen tartrate,
- Mixtures of phthalic acid and its salts, in particular the potassium salts, especially potassium hydrogen phthalate,
- Mixtures of lactic acid and its salts, in particular lactic acid/sodium lactate mixtures,
- Mixtures of gluconic acid and its salts, in particular gluconic acid/sodium gluconate mixtures,
- Mixtures of succinic acid and its salts, in particular the sodium salts, especially sodium hydrogen succinate and disodium succinate, and
- Mixtures of malic acid and its salts, in particular the sodium salts, especially sodium hydrogen malate and disodium malate,

15. Process for the non-oxidative dyeing of keratinous fibres, in particular human hair, according to one of the preceding claims, **characterized in that** the keratinous fibres have not been treated with an oxidizing agent and not with a keratin-reducing agent for a period of up to 7 days prior to the application of the dyeing composition.

## Revendications

1. Composition cosmétique pour la teinture sans agent oxydant et sans mordant de fibres kératiniques, en particulier de cheveux humains, avec des colorants naturels, contenant :
i. au moins un extrait tinctorial d'un bois de couleur,
ii. de l'eau,
iii. un système tampon choisi parmi un mélange d'un acide moyennement fort ou faible avec sa base conjuguée ou correspondante (ou le sel respectif) et un mélange d'une base moyennement forte ou faible avec son acide conjugué ou correspondant,
iv. ladite composition ayant un pH dans la gamme de 3 à 5,9, mesuré à 20°C,
ladite composition ne contenant pas d'agent de décapage choisi parmi les sels de magnésium, de calcium, d'aluminium, de métaux de transition et de lanthanides, pas de cystéine, pas de N-acétylcystéine, pas de précurseur de colorant d'oxydation et pas de substances grasses présentant une solubilité dans l'eau d'au plus 0,01 g/l à 20°C.

2. Procédé de teinture sans oxydant et sans mordant de fibres kératiniques, en particulier de cheveux humains, avec des colorants naturels, dans lequel une composition de teinture contenant :
i. au moins un extrait tinctorial d'un bois de couleur,
ii. de l'eau,
iii. un système tampon choisi parmi un mélange d'un acide moyennement fort ou faible avec sa base conjuguée ou correspondante (ou le sel respectif) et un mélange d'une base moyennement forte ou faible avec son acide conjugué ou correspondant,
iv. dans laquelle la composition de coloration a un pH dans la plage de 3,0 à 10,5, mesuré à 20°C,
ladite composition de teinture ne contenant pas de mordant choisi parmi les sels de magnésium, de calcium, d'aluminium, de métaux de transition et de lanthanides, pas de cystéine, pas de N-acétylcystéine, pas de précurseur de colorant d'oxydation et pas de substances grasses qui présentent une solubilité dans l'eau d'au plus 0,01 g/l à 20°C,
est appliqué sur les fibres kératiniques, en particulier sur les cheveux humains, et est rincé à l'eau après un temps d'action de 30 secondes à 60 minutes, de préférence de 5 à 45 minutes, de manière particulièrement préférée de 15 à 30 minutes, le procédé ne comprenant pas l'application d'un mordant choisi parmi les sels de magnésium, de calcium, d'aluminium, de métaux de transition et de lanthanides.

3. Composition selon la revendication 1 ou procédé selon la revendication 2, **caractérisée en ce que** ledit au moins un extrait de bois coloré colorant i. est choisi parmi extraits du bois de *Haematoxylum brasiletto L.* (arbre à bois sanglant brésilien), *Haematoxylum campechianum* (arbre à bois sanglant, arbre à bois bleu, arbre à camphre), et *Maclura tinctoria* (mûrier teinturier).

4. Composition ou procédé selon l'une quelconque des revendications précédentes, caractérisé(e) en ce que ledit au moins un extrait de bois coloré i contient un polyphénol choisi parmi la brésiline, la brasiline, l'hématoxyline, l'hématéine, la maclurine, le kämpferol, la morine ainsi que leurs mélanges.

5. Composition ou procédé selon l'une quelconque des revendications précédentes, caractérisé(e) en ce que la composition contient, dans chaque cas par rapport à son poids, l'au moins un extrait de bois coloré colorant i dans une quantité de 0,1 à 20 % en poids, de préférence de 0,2 à 10 % en poids, de manière particulièrement préférée de 0,5 à 5 % en poids, de manière exceptionnellement préférée de 1 à 4 % en poids, de manière encore plus préférée de 2 à 3 % en poids.

6. Composition ou procédé selon l'une quelconque des revendications précédentes, caractérisé(e) en ce que la composition contient, dans chaque cas par rapport à son poids, au moins un polyphénol, dans chaque cas en une quantité de 0,05 à 10 % en poids, de préférence de 0,1 à 5 % en poids, de manière particulièrement préférée de 0,5 à 4 % en poids, de manière exceptionnellement préférée de 1 à 2 % en poids.

7. Composition ou procédé selon l'une quelconque des revendications précédentes, caractérisé(e) en ce que la composition contient, dans chaque cas sur la base de son poids, au moins un polyphénol choisi parmi la brasiline, la brésiline, l'hématoxyline, l'hématéine, la maclurine, le combattantol, la morine ainsi que leurs mélanges, dans chaque cas en une quantité de 0,05 à 10 % en poids, de préférence de 0,1 à 5 % en poids, de manière particulièrement préférée de 0,5 à 4 % en poids, de manière particulièrement préférée de 1 à 2 % en poids.

8. Composition ou procédé selon l'une quelconque des revendications précédentes, caractérisé(e) en ce que, lorsque le polyphénol est choisi parmi l'hématoxyline, aucun autre polyphénol n'est présent en dehors de l'hématoxyline et de son produit de fermentation, l'hématéine.

9. Composition ou procédé selon l'une quelconque des revendications précédentes, **caractérisé par** une teneur en eau de 40 à 98 % en poids, de préférence de 50 à 97 % en poids, de manière particulièrement préférée de 60 à 85 % en poids, dans chaque cas par rapport à leur poids.

10. Composition ou procédé selon l'une quelconque des revendications précédentes, caractérisé(e) en ce qu'elle ne contient pas de métabisulfite.

11. Composition selon l'une quelconque des revendications précédentes, **caractérisée en ce que** la composition présente un pH dans la plage de 4 à 5,5, de préférence dans la plage de 4,5 à 5,2, de manière particulièrement préférée dans la plage de 4,8 à 5,0, dans chaque cas mesuré à 20°C.

12. Composition selon l'une quelconque des revendications précédentes, **caractérisée en ce que** le système tampon est choisi parmi
- Mélanges d'acide citrique et de ses sels, notamment les citrates de métaux alcalins, en particulier les sels de sodium, notamment le citrate trisodique,
- Mélanges d'acide tartrique et de ses sels, notamment les tartrates de métaux alcalins, en particulier les sels de potassium, notamment l'hydrogénotartrate de potassium,
- Mélanges d'acide phtalique et de ses sels, en particulier les sels de potassium, notamment l'hydrogénophtalate de potassium,
- Mélanges d'acide lactique et de ses sels, notamment les mélanges d'acide lactique et de lactate de sodium,
- Mélanges d'acide gluconique et de ses sels, notamment les mélanges acide gluconique/gluconate de sodium,
- Mélanges d'acide succinique et de ses sels, en particulier les sels de sodium, notamment l'hydrogénosuccinate de sodium et le succinate de disodium, et
- Mélanges d'acide malique et de ses sels, en particulier les sels de sodium, notamment l'hydrogénomalate de sodium et le malate de disodium.

13. Procédé selon l'une quelconque des revendications précédentes, **caractérisé en ce que** la composition colorante présente un pH dans la plage de 4,0 à 10,0, de préférence dans la plage de 5,5 à 9,5, de manière particulièrement préférée dans la plage de 7,0 à 8,0, dans chaque cas mesuré à 20°C.

14. Procédé selon l'une quelconque des revendications précédentes, **caractérisé en ce que** le système tampon est choisi parmi
- Mélanges de sels de phosphate et d'hydrogénophosphate, en particulier les sels de métaux alcalins, en particulier les sels de sodium, notamment les mélanges de phosphate trisodique/hydrogénophosphate disodique et les mélanges d'hydrogénophosphate disodique/dihydrogénophosphate de sodium,
- Mélanges de sels de carbonate et d'hydrogénocarbonate, notamment les sels de métaux alcalins, en particulier les sels de sodium, notamment les mélanges de carbonate disodique/hydrogénocarbonate de sodium,
- Mélanges d'acide citrique et de ses sels, notamment les citrates de métaux alcalins, en particulier les sels de sodium, notamment le citrate trisodique,
- Mélanges d'acide tartrique et de ses sels, notamment les tartrates de métaux alcalins, en particulier les sels de potassium, notamment l'hydrogénotartrate de potassium,
- Mélanges d'acide phtalique et de ses sels, en particulier les sels de potassium, notamment l'hydrogénophtalate de potassium,
- Mélanges d'acide lactique et de ses sels, notamment les mélanges d'acide lactique et de lactate de sodium,
- Mélanges d'acide gluconique et de ses sels, notamment les mélanges acide gluconique/gluconate de sodium,
- Mélanges d'acide succinique et de ses sels, en particulier les sels de sodium, notamment l'hydrogénosuccinate de sodium et le succinate de disodium, et
- Mélanges d'acide malique et de ses sels, en particulier les sels de sodium, notamment l'hydrogénomalate de sodium et le malate de disodium,

15. Procédé de coloration non oxydante des fibres kératiniques, en particulier des cheveux humains, selon l'une quelconque des revendications précédentes, **caractérisé en ce que** les fibres kératiniques n'ont pas été traitées par un agent oxydant et n'ont pas été traitées par un agent réducteur de kératine pendant une période allant jusqu'à 7 jours avant l'application de la composition colorante.
